# FASCICULE DE BREVET EUROPEEN

(11) **EP 0 912 466 B1**
(45) Date de publication et mention de la délivrance du brevet: **04.09.2002**
(21) Numéro de dépôt: 97923142.0
(22) Date de dépôt: 06.05.1997
(51) Int. Cl.: C07B 39/00, C07C 17/20, C07C 22/08

(54) **PROCEDE DE SYNTHESE DE COMPOSES HYDROCARBONES FLUORES SUR AU MOINS UN CARBONE D'UNE CHAINE ALCOYLE**
VERFAHREN ZUR HERSTELLUNG VON AUF MINDESTENS EINEM KOHLENSTOFFATOM IN EINER ALKYLKETTE FLUORIERTEN KOHLENWASSERSTOFFEN
METHOD FOR SYNTHESIS OF HYDROCARBON COMPOUNDS CONTAINING FLUORINE ON AT LEAST ONE ALKYL CHAIN CARBON

(30) Priorité: 10.05.1996 FR 9605859
(43) Date de publication de la demande: 06.05.1999
(73) Titulaire: RHODIA CHIMIE, 92408 Courbevoie Cédex (FR)
(72) Inventeur: SAINT-JALMES, Laurent, F-69330 Meyzieu (FR)
(74) Mandataire: Ricalens, François
(86) Numéro de dépôt international: FR9700803
(87) Numéro de publication internationale: WO97043231

(56) Documents cités:
- EP-A- 0 005 810
- EP-A- 0 729 930
- DATABASE WPI Section Ch, Week 8430 Derwent Publications Ltd., London, GB; Class E14, AN 84-185448 XP002022623 & JP 59 104 328 A (HODOGAYA CHEM IND KK) , 16 Juin 1984
- J. ORG. CHEM. (JOCEAH,00223263);79; VOL.44 (22); PP.3872-81, UNIV. SOUTHERN CALIFORNIA;INST. HYDROCARBON CHEM.; LOS ANGELES; 90007; CA; USA, XP002022622 OLAH G A ET AL: "Synthetic methods and reactions. 63. Pyridinium poly(hydrogen fluoride) (30% pyridine-70% hydrogen fluoride): a convenient reagent for organic fluorination reactions"

## Description

La présente invention a pour objet un procédé utile pour la synthèse de composés hydrocarbonés fluorés sur un carbone d'une chaîne alcoyle par échange entre un halogène de rang plus élevé et le fluor au moyen d'un réactif contenant du fluor au moins partiellement sous forme de sels complexes. Elle concerne plus particulièrement un procédé permettant l'obtention de dérivés fluorés sur un atome de carbone portant des groupes présentant une instauration ou des doubles liaisons.

Les composés fluorés sont en général difficiles d'accès. La réactivité du fluor est telle qu'il est difficile voire impossible d'obtenir directement les dérivés fluorés.

Une des techniques les plus employées pour fabriquer le dérivé fluoré consiste à faire réagir un dérivé halogéné, en général chloré, pour échanger l'halogène avec un fluor minéral, en général un fluorure de métal alcalin, en général de poids atomique élevé.

En général, le fluorure utilisé est le fluorure de potassium qui constitue un compromis économique satisfaisant.

Dans ces conditions, de nombreux procédés tels que par exemple ceux décrits dans le certificat d'addition N° 2 353 516 et dans l'article Chem. Ind. (1978) - 56 ont été décrits et mis en oeuvre industriellement pour obtenir des fluorures d'aryle, aryles sur lesquels sont greffés des groupements électroattracteurs.

Sauf dans les cas où le substrat est particulièrement adapté à ce type de synthèse, cette technique présente des inconvénients dont les principaux sont ceux que l'on va analyser ci-après.

La réaction nécessite des réactifs comme les fluorures de métal alcalin tels que le fluorure de potassium qui sont rendus relativement chers par les spécifications auxquels ils doivent répondre pour être adaptés à ce type de synthèse ; ils doivent être très purs, secs et sous une forme physique adaptée.

En outre cette réaction ne marche pas pour toute une classe de produits notamment ceux portant sur le carbone halogénophore (c'est à dire le carbone portant le ou les halogènes destinés à être échangés avec le fluor).

Il est aussi utilisé des réactifs tels que l'acide fluorhydrique liquide, dilué par des solvants aprotiques dipolaires ou gazeux (voir Database WPI, section Ch, Week 8430, Derwent publ, Ltd, AN84-185448 & JP-A-5910432). Toutefois l'acide fluorhydrique est un réactif trop puissant et conduit souvent à des réactions de polymérisation indésirées ou à des goudrons.

Dans ce cas, et notamment dans le cas où l'on désire des dérivés fluorés sur un carbone de type alcoyle (y compris aralcoyle) appauvri en électron par la présence de groupes de type électroattracteur, l'homme de métier se trouve devant une alternative dont les termes ne sont guère encourageants ; ou bien l'on choisit des conditions très dures et l'on obtient surtout des goudrons, ou bien l'on se place dans des conditions réactionnelles douces et l'on retrouve, dans le meilleur des cas le substrat inchangé. Enfin il convient de signaler que certains auteurs ont proposé de réaliser des échanges en utilisant comme réactif des sels de l'acide fluorhydrique en présence d'éléments lourds sous forme d'oxydes ou de fluorures. Parmi les éléments utilisés, il convient de citer l'antimoine et les métaux lourds tels que l'argent ou le vif-argent (mercure); cf. en particulier Olah et Al (J.O.C., vol 44, no 22, 1979, pp. 3872-3880) qui mentionne l'échange chlore fluor en présence d'oxyde mercureux.
Il a été proposé dans la demande (1) EP-A-5810 d'utiliser des fluorhydrate et de poly fluorhydrate d'amine pour l'echange d'un monohalogéne avec le fluor mais la réaction semble ne pas fonctionner avec les polyhalogénés sur le même carbone.
Plus récemment, la Demanderesse a déposé la demande EP-A-729 930 visant l'activation par les chalcogènes des carbones chlorés en vue de l'échange au moyen d'un réactif associant base de Brönstedt et acide fluorhydrique.

Un autre problème réside dans la sélectivité de la réaction : lorsque il y a plusieurs halogènes à échanger sur le même carbone, il est souvent difficile de n'en échanger qu'une partie.

C'est pourquoi un des buts de la présente invention est de fournir un procédé qui soit susceptible de réaliser l'échange entre d'une part les halogènes lourds tels que le chlore et d'autre part le fluor en améliorant significativement la spécificité de la réaction.
Un autre but de la présente invention est de fournir un procédé qui soit susceptible de réaliser l'échange entre d'une part les halogènes lourds tels que le chlore et d'autre part le fluor en utilisant des conditions réactionnelles particulièrement douces. Un autre but de la présente invention est de fournir un procédé qui permette d'utiliser une source de fluorure dont la morphologie soit moins critique.

Un autre but de la présente invention est de fournir un procédé qui permette de n'échanger qu'un atome d'halogène sur deux ou sur trois possibles.

Un autre but de la présente invention est de fournir un procédé qui permette de n'échanger que deux atomes d'halogène sur trois possibles.

Un autre but de la présente invention est de fournir un procédé qui permette de n'échanger les molécules ou les atomes que dans la mesure où cela permet d'obtenir des atomes de carbone qui ne soient porteurs que d'un atome de fluor concomitamment avec un ou deux autres halogènes distincts du fluor.

Un autre but de la présente invention est de fournir un procédé qui permette de n'échanger les molécules ou les atomes que dans la mesure où cela permet d'obtenir des atomes de carbone qui ne soient porteurs que de deux atomes de fluor concomitamment avec un autre halogène distinct du fluor.

Un autre but de la présente invention est de fournir un procédé qui permette d'éviter l'usage de forte quantité de métaux réputés coûteux ou toxiques tels que le mercure et/ou l'argent.

Un autre but de la présente invention est de fournir un procédé qui permette de réduire les quantités de métaux réputés coûteux ou toxiques tels que le mercure et/ou l'argent, de manière que le rapport molaire entre le métal et le substrat dont les atomes d'halogène sont à échanger, se situe à une valeur au plus égale à 0,5, avantageusement à 0,2, de préférence à 0,1.

Un autre but de la présente invention est de fournir un procédé qui permette d'éviter complètement l'utilisation de métaux réputés coûteux ou toxiques tels que le mercure et/ou l'argent, de manière à n'ajouter au mélange réactionnel aucun des éléments cités ci-dessus ; en d'autres termes que les concentrations en chacun desdits métaux ne dépassent pas les valeurs de 10⁻³ M; avantageusement 10⁻⁴ M, de préférence de 10⁻⁵ M.

Ces buts et d'autres qui apparaîtront par la suite sont atteints au moyen d'un procédé utile pour la synthèse de dérivés fluorés par échange entre un halogène de rang plus élevé et le fluor, caractérisé par le fait qu'il comporte l'étape de soumettre un substrat comportant au moins un carbone halogénophore d'hybridation sp³ porteur d'au moins deux halogènes dont au moins un est un halogène de nombre atomique supérieur à celui du fluor, lequel carbone halogénophore est relié à au moins un atome de faible hybridation porteur d'une insaturation, est soumis à l'action d'un réactif comportant au moins un composé défini choisi parmi ceux constitués par l'association d'une base de Bronstedt avec un nombre défini n d'acide fluorhydrique, n étant au mois égal à 3 et au plus à 20, avantageusement à 15, de préférence à 10.
La présente invention ne vise pas le cas où ledit carbone halogénophore est porteur de chalcogène.

Outre le cas où ledit atome de faible hybridation porteur d'une insaturation est engagé dans une liaison carbone-carbone (acétylénique, de préférence éthylénique laquelle liaison éthylénique est avantageusement engagé dans un cycle à caractère aromatique), on peut indiquer à titre d'enseignement par l'exemple que, avantageusement, ledit atome de faible hybridation porteur d'une insaturation est un atome engagé dans une des doubles liaisons [où *C est le carbone halogénophore] suivantes :

| atome de faible degré de facilité de remarques hybridation et l'échange la insaturation dont réaction (facile = 1 ; il est porteur moins facile =2 mais plus sélective; relativement difficile =3) | | |
|---|---|---|
| -*C-CR"=NR' | 2 | avec HF constitue déjà un milieu HF base [la séquence peut même se trouver dans des pyridines substituées]* |
| -*C-CR"=S' | 1 | |
| -*C-C=N-NH-R' | 2 | avec HF constitue déjà un milieu HF base* |
| -*C-CR"=N-O-R' | 2 | avec HF constitue déjà un milieu HF base* |
| -*C-CR"=PR' | 2 | avec HF constitue déjà un milieu HF base* |
| -*C-N=NR' | 2 | composés parfois fragiles ce qui limite le domaine des conditions opératoires acceptables |
| -*C-CF=CF₂ | 2 | |
| -*C-CR="O | 3 | Réaction difficile |
| -*C-N=O | 2 | peut donner lieu à de mélanges très complexes |

| | | |
|---|---|---|
| * toutefois la réaction étant en général menée avec un très large excès de réactif il est préférable d'utiliser une base distincte pour réaliser l'excès de réactif (correspondant ou non à la stoechiométrie) surtout quand un réactif fort est nécessaire. Il est quasiment impossible de donner la stoechiométrie de la réaction d'échange ; nonobstant à titre indicatif, si l'on pose que chaque composé HF base n'échange qu'un fluor, alors il est très souhaitable d'utiliser au moins a quantité stoechiométrique, avantageusement au moins une fois et demie, de préférence au moins cinq fois, plus généralement au moins 10 fois. Il n'y a comme limite supérieure qu'une limite économique en général environ 100 (dans la présente description le terme "environ" est employé pour mettre en exergue le fait que, lorsque le, ou les, chiffres les plus à droite d'un nombre sont des zéros, ces zéros sont des zéros de position et non des chiffres significatifs, sauf bien entendu s'il en est précisé autrement). | | |

Dans la formule I relative aux substrats préférés R" correspond à R₁₀, cependant que R' correspond à R₅.

En effet au cours de l'étude qui a mené à la présente invention, il a été montré que certains atomes de carbone (qualifié dans la présente description d'halogénophore car porteur d'halogène) portant des groupes électroattracteurs par effet inductif, sous la condition qu'au moins un des substituants du carbone halogénophore fût porteur d'une insaturation stable dans les conditions opératoires en que ledit carbone halogénophore se situât en α, étaient susceptibles de réagir avec un réactif du type ci-dessus.
Rappelons que la présente invention ne vise pas le cas où ledit carbone halogénophore est porteur de chalcogène.

La température de réaction varie du point de fusion du mélange réactionnel à son point de décomposition ou d'ébullition, en général de 0°C à 150°C, avantageusement de 20 à 100°C.

On travaille en général à la pression atmosphérique, mais il est possible de travailler à des pressions pouvant atteindre 20 10⁵ Pascals.

Parmi les bases préférées, on peut citer celles qui sont des dérivés trivalents hydrocarbonés des éléments de la colonne VB, avantageusement de période d'un rang au moins égal à la deuxième et en général inférieure à la sixième, de la classification périodique des éléments (supplément au Bulletin de la Société Chimique de France Janvier 1966 N°1). Outre ceux qui sont détaillés par la suite on peut donner comme exemples de tels composés les dérivés trivalents, qui lorsqu'ils sont trisubstitués sont en fait des pnictines, pnictines qui font l'objet d'une description plus détaillée ci-après.

Parmi lesdits dérivés hydrocarbonés des éléments de la colonne V les préférés sont ceux qui dérivent des pnictures d'hydrogène par substitution totale ou partielle de l'hydrogène par des restes hydrocarbonés qui peuvent être reliés à l'atome de la colonne V B par une double (comme dans les imines) ou une triple liaison (comme dans les nitriles).

Toutefois les dérivés hydrocarbonés des éléments de la colonne V dérivent avantageusement des pnictures d'hydrogène par substitution totale ou partielle de l'hydrogène par des restes hydrocarbonés monovalents avantageusement par des alcoyles [dans la présente description **ALCO-*yle*** est pris dans son sens étymologique de reste hydrocarboné d'un **ALCO-*ol*** après ignorance de la fonction alcool (ou ol)]; ces composés alcoylés seront, par analogie avec le terme de pnicture, désignés dans la présente description, sous le terme de pnictines.

Ainsi dans le cas de l'azote la substitution du nitrure d'hydrogène (ammoniac) donne les amines, dans le cas du phosphore la substitution du phosphure d'hydrogène donne les phosphines, dans le cas de l'arsenic la substitution de l'arséniure d'hydrogène donne les arsines et dans le cas de l'antimoine la substitution de l'antimoniure (ou stibiure) d'hydrogène donne les stibines. Ils sont avantageusement choisis parmi les dérivés hydrocarbonés du phosphore tels que les phosphines.

Par ailleurs, plus la base est faible et plus elle est molle, meilleur et plus complet est l'échange. Ainsi les amines primaires, secondaires et de préférence tertiaires conduisent à des réactifs contenant peu de groupes HF (au plus 5, en général moins) et moins puissants que les bases de type hétérocycle aromatique dont le ou au moins l'un des hétéroatomes est choisi dans la colonne V.
Ces composés formés d'une base et d'un nombre discret d'unité HF seront désignés ci-après sous le terme de complexe(s) "HF Base" ou "Base HF".
La présente invention ne vise pas les échanges avec les fluorures métalliques (notamment alcalins tel que KF, CsF ..) cela peut s'exprimer par le fait que la quantité [(exprimée en équivalent) de cation(s) (alcalins, ammoniums)], doit être au moins égale à une fois (avantageusement au moins à 4/3 de fois, de préférence à 2 fois environ) celle de l'hydrogène sous forme de proton libre, d'acide halohydrique dégagé, ou de complexes "base-HF" y compris "F⁻(HF)".

On peut donner la règle empirique suivante : si les bases forment des composés définis de plus de 5 HF par fonction basique (le paradigme des réactifs forts est le composé défini pyridine, 10 HF), alors c'est un réactif puissant susceptible d'échanger deux halogènes lourds sur le même carbone dans des conditions très douces et même trois dans des conditions un peu plus dures (température et pression). Sinon il s'agit d'un réactif plus sélectif qui n'échange, en général, que pour donner un seul fluor sur un carbone dans des conditions douces et deux fluors sur le carbone dit halogénophore dans des conditions plus sévères. Cette invention est surtout intéressante pour remplacer des chlores par des fluors.
Ainsi, les réactions d'échange sont essentiellement successives (en effet chaque atome de fluor supplémentaire sur le carbone halogénophore ralentit l'échange des atomes d'halogène plus lourds que le fluor avec ce dernier), ce qui permet de réaliser un échange sélectif ou complet, en jouant sur les conditions opératoires et sur le choix des réactifs. Comme il est en général possible de trouver des conditions où la réaction d'échange s'arrête avant que la totalité des halogènes plus lourds que le fluor ne soit remplacé par ce dernier, il s'ensuit qu'une sélectivité à deux volets est possible. D'une part il est possible de n'échanger qu'un nombre limité des halogènes plus lourds que le fluor et d'autre part il est possible de traiter un mélange déjà partieilement fluorés et de ne toucher de manière significative que les molécules n'ayant pas atteint le nombre désiré d'atome de fluor.
En général la facilité d'échange d'un atome d'halogène plus lourd que le fluor avec ce dernier, croît avec son nombre atomique.
Bien évidemment on peut jouer sur la stoechiométrie et son excès pour limiter le nombre des atomes d'halogène échangés par molécule.

Il peut y avoir plusieurs atomes de carbone halogénophore par molécule. Il est préférable que deux atomes halogénophores n'interfèrent pas l'un avec l'autre.

On donnera ci-après une typologie des atomes de carbone, voire des molécules, les plus aptes à échanger leurs halogènes lourds avec le fluor sous l'action des réactifs ci-dessus. Chaque caractéristique ci-après rend plus vif l'intérêt de l'invention pour lesdits carbones.

Ainsi il est particulièrement intéressant que l'éventuelle liaison résiduelle du carbone halogénophore soit avantageusement une liaison avec un groupe choisi parmi les groupes électroattracteurs par effet inductif. Ledit groupe choisi parmi les groupes électroattracteurs est avantageusement un halogène.

Ainsi il est également particulièrement intéressant que l'éventuelle liaison résiduelle du carbone halogénophore soit avantageusement une liaison avec un autre atome de faible hybridation porteur d'une insaturation.
Ladite insaturation dont ledit atome de faible hybridation est porteur est avantageusement choisie de manière que les réactions d'addition ou de polymérisation soient peu favorisées. Ainsi une insaturation aromatique est particulièrement bien adaptée.

L'invention est particulièrement utile quand ledit carbone halogénophore de départ porte au moins deux halogènes de nombre atomique supérieur à celui du fluor.

Ainsi que l'on l'exposera plus tard, l'invention est particulièrement intéressante lorsque ledit carbone halogénophore est trihalogénométhyle, c'est-à-dire qu'il porte trois halogènes choisis avantageusement parmi le chlore et le fluor.
Ainsi, pour paraphraser ce qui précède, et quitte à être superfétatoire, on peut indiquer que les substrats peuvent avantageusement être des molécules de formule I :

R-CXX'-Z(R₁₀)ᵣ=Z'(R₁₁)ₛ-(R₅)ₜ (I)

Avec R choisi parmi reste hydrocarbonés (notamment aryle ou alcoyle), les halogènes, les groupes électroattracteurs (de préférence par effet inducteur) ;
avec X et X' semblables ou différents, choisis parmi les halogènes, de préférence le chlore (avec bien entendu la condition que R, X et X' ne peuvent être simultanément fluor et que l'un d'entre eux représente au moins un halogène plus lourd que le fluor à échanger avec le fluor) ;
Z est choisi parmi les métalloïdes trivalents avec r égal à zéro ou tétravalents avec r égal à 1 (respectivement phosphore et avantageusement azote d'une part et carbone d'autre part, de préférence carbone) ;
et Z' est choisi parmi les métalloïdes avantageusement les chalcogènes (avec s et t égaux à zéro), l'azote et le phosphore (avec s égal à zéro) et le carbone avec s et t égal à1);
r, s, et t peuvent prendre les valeurs zéro ou un, selon ce que représente Z et Z'.

De manière surprenante R peut être hydrogène et donner lieu à un échange facile surtout lorsque le composé est de formule deux, de préférence lorsque Ar est homocylique.
R peut être aussi de type -Z(R₁₀)ᵣ=Z'(R₁₁)ₛ-(R₅)ₜ y compris de type Ar (R₁₁)ₛ. pour donner ou non une molécule symétrique.
R₅ peut être hydrogène ou un quelconque radical, avantageusement hydrocarboné (c'est-à-dire contenant carbone et hydrogène).
R₁₀ peut prendre indépendamment les mêmes valeurs que R₅
R₁₁ peut prendre indépendamment les mêmes valeurs que R₅
toutefois selon la présente invention R₁₀ et R₅ sont avantageusement reliés pour former un cycle aromatique, ce qui implique alors que Z est carbone.
La formule (I) devient alors

R--CXX'-Ar (R₁₁)ₛ. (formule II)

Ou Ar représente un cycle aromatique éventuellement substitué. Il représente notamment un cycle benzénique (tel qu'un phényle ou un naphtyle) éventuellement substitué, un hétérocycle éventuellement substitué,
R₁₁ représentant alors l'éventuel substituant en ortho du carbone halogénophore (CXX').
Les composés où Ar présente cinq chaînons, de préférence avec deux hétéroatomes (il est souhaitable d'avoir deux atomes d'azote) sont d'un intérêt particulier ;
Lorsque Z(R₁₀)ᵣ=Z'(R₁₁)ₛ-(R₅)ₜ est électroattracteur (comme dans le cas des hétérocycles à six chaînons comme la pyridine), il convient de noter que l'échange est plus difficile, surtout pour le troisième atome de fluor sur le même carbone. Ceci est particulièrement avantageux dans le cas d'un échange partiel sélectif.
Chaque radical R et R₅ R₁₀ et R₁₁comporte habituellement au plus 30 (dont au plus 20 atomes de carbone), avantageusement 20 (dont au plus 15 atomes de carbone), de préférence 15 atomes de carbone et/ou d'azote (dont au plus 12 atomes de carbone). Le nombre total de carbones des molécules substrats dépasse rarement 50, et est avantageusement d'au plus 30.
R₅ représente un cycle phényle éventuellement substitué, un hétérocycle éventuellement substitué, avantageusement à cinq chaînons, de préférence à deux hétéroatomes (il est souhaitable d'avoir deux atomes d'azote) ;
Par électrodonneur ou faible électroattracteur, il convient de comprendre aussi attracteur ou moins attracteur qu'un dichlorophényle (définition qui convient aussi pour aryle non électroattracteur ou pour un radical arylique riche en électron). Par différence, on déduit la définition "d'électroattracteur" ou de "significativement électroattracteur".
Pour aider l'homme de métier à déterminer les conditions à choisir suivant les cas rencontrés, on trouvera ci-après des règles empiriques utilisables dans la majorité des situations rencontrées.
Si l'on désire réaliser des échanges complets il est souhaitable d'utiliser des réactifs forts dans des conditions dures ou très dures. A l'inverse des réactifs faibles dans des conditions douces conduisent à des échanges très sélectifs en général à des monoéchanges (conduisant à des produits monofluorés sur le carbone halogénophore). Réactif faible et condition dures ainsi que réactif fort et conditions douces conduisent en général à des résultats intermédiaires.
Ces règles sont à moduler par l'aptitude des substrats à être substitués. Plus les substituants du carbone halogénophore sont globalement donneurs plus le substrat a des facilités à s'échanger (c'est-à-dire plus le nombre final de fluor sur le carbone halogénophore sera facile à atteindre).
Le paradigme des réactifs faibles est le composé défini triéthylamine, 3 HF
Le paradigme des réactifs forts est le composé défini pyridine, 10 HF
conditions douces : θ = point de fusion à 50°C au plus ;
conditions dures : 50°C à 100°C (ou au point d'ébullition s'il est plus bas à la pression considérée) ; conditions très dures :θ = 100 à 150°C et le cas échéant pressions supérieures à l'atmosphérique ;
la sélectivité vient du fait que en utilisant les réactifs de la présente invention, les échanges sont successifs et plus il y a de fluor sur le carbone Halogénophore plus la réaction est difficile et plus elle est lente.
Dans le cas où la formule (I) X et X' représentant des halogènes plus lourds que le fluor, on peut expliciter les équations réactionnelles
Réaction conduisant à un fluor sur le carbone Halogénophore :

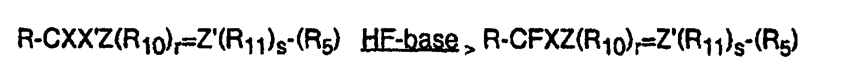

Réactions conduisant à deux fluors sur le carbone Halogénophore :

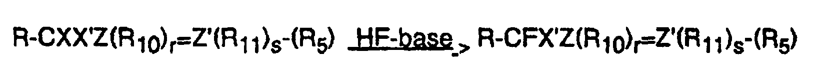

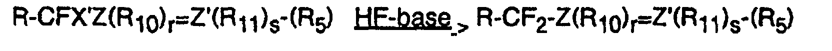

Et avec R représentant un halogène plus lourd que le fluor

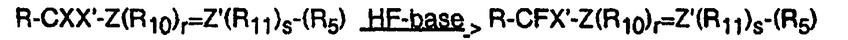

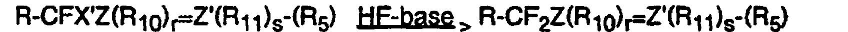

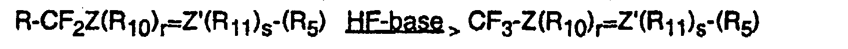

Les exemples non limitatifs suivants illustrent l'invention.

### Préparation des Solutions HF - base - Mode opératoire général

Les différents milieux HF - base sont synthétisés comme suit :

A x moles d'une base organique (pyridine, triéthylamine, dioxane, ...) ou minérale (KF, bu₄NF, ...) sous agitation (éventuellement refroidies à -20°C) sont ajoutées Y moles d'acide fluorhydrique anhydre goutte à goutte. Après addition de l'acide fluorhydrique anhydre, le milieu réactionnel est réchauffé à température ambiante et utilisé sans aucun traitement. Le complexe HF - base a donc la structure (HF)_{y} - Baseₓ.

Après la réaction, lorsque le brut de fluoration est traité par une phase organique anhydre non miscible avec le milieu HF-Base considéré, mais apte à dissoudre le produit d'arrivée (Par exemple uniquement du CH₂Cl₂ (sans glace ou eau)) 2 phases sont obtenues : la phase la moins polaire (Par exemple CH₂Cl₂) qui contient le produit obtenu après échange, et la phase la plus polaire "HF - base" qui peut être alors recyclée, après une éventuelle remise au titre initial (en HF) et élimination de l'acide halohydrique dégagé par la réaction (Par exemple par distillation). Ce recyclage est propre au procédé selon la présente invention et est un avantage supplémentaire du procédé.

### Exemple N°1 Echange Cl-F à partir du trichlorométhylbenzène

### 1a) Echange d'un atome de chlore

### Equation réactionnelle

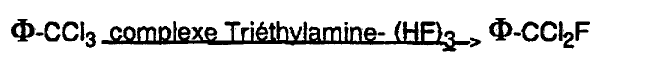

Mode opératoire utilisé :
1,95 g (0,01 mole) du trichloro-méthyl benzène est additionné à 21 g (0,13 mole) de complexe [HF]₃- triéthylamine à 20°C.

Le milieu réactionnel est ensuite chauffé et agité à 70°C pendant 10h.

Le brut réactionnel est alors versé sur un mélange de CH₂Cl₂ (200 ml) et de glace (200g).

La phase organique est lavée 4 fois par 50 ml d'eau, séchée sur sulfate de magnésium. Le solvant (CH₂Cl₂) est évaporé et le résidu est analysé par chromatographie en phase gazeuse et les produits formés sont identifiés par RMN ¹⁹F.
On obtient alors un mélange de 2 produits :

| | |
|---|---|
| Φ-CCl₂F | 86% |
| Φ-CClF₂ | 8% |

Le taux de transformation du trichlorométhylbenzène est de 100%. Un mono échange d'atome de chlore est donc réalisé dans ces conditions réactionnelles.

### 1b) Echange de 2 atomes de chlore

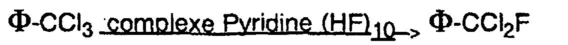

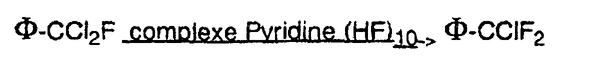

Mode opératoire utilisé
1,95 g (0,01 mole) du trichloro-môthyl benzène est additionné à 11,2g (0,04 mole) de complexe [HF]₁₀- pyridine 1 (préparé à partir de 77,3 g de pyridine et 200 g d'acide fluorhydrique anhydre) refroidi à 0°C.

Le milieu réactionnel est ensuite ramené à température ambiante et agité pendant 2h.

Le brut réactionnel est alors versé sur un mélange de CH₂Cl₂ (200 ml) et de glace (200g).

La phase organique est lavée 4 fois par 100 ml d'eau, séchée sur sulfate de magnésium. Le solvant (CH₂Cl₂) est évaporé pour fournir une huile qui est analysée par chromatographie en phase gazeuse, la structure des produits étant obtenue par RMN ¹⁹F.
On obtient alors un mélange de 2 produits :

| | |
|---|---|
| Φ-CClF₂ | 92% |
| Φ-CF₃ | 8% |

Le taux de transformation du trichlorométhylbenzène est de 100%.
Un di-échange est donc réalisé dans ces conditions réactionnelles.

### 1c) Echange de trois atomes de chlores

### Equations réactionnelles

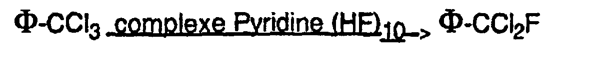

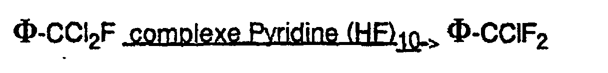

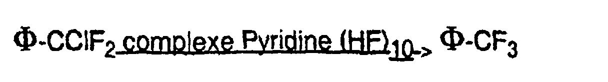

Mode opératoire utilisé
1,95 g (0,01 mole) du trichloro-méthyl benzène est additionné à 11,2 g (0,04 mole) de complexe [HF]₁₀- pyridine 1 (préparé à partir de 77,3 g de pyridine et 200 g d'acide fluorhydrique anhydre) refroidi à 0°C.
Le milieu réactionnel est ensuite chauffé à 50°C et agité pendant 24 heures.
Le brut réactionnel est alors versé sur un mélange de CH₂Cl₂ (200 ml) et de glace (200g).
La phase organique est lavée 4 fois par 100 ml d'eau, séchée sur sulfate de magnésium. Le solvant (CH₂Cl₂) est évaporé pour fournir une huile qui est analysée par chromatographie en phase gazeuse, la structure des produits étant obtenue par RMN ¹⁹F.
On obtient alors un mélange de 2 produits :

| | |
|---|---|
| Φ-CCIF₂ | 15% |
| Φ-CF₃ | 84% |

Le taux de transformation du trichlorométhylbenzène est de 100%.
Un tri-échange est donc réalisé majoritairement dans ces conditions réactionnelles.
Les résultats obtenus à partir de trichlorométhylbenzène sont rassemblés dans le tableau suivant.

| Milieu HF-Base utilisé | Durée | température | Φ-CCl₃ | Φ-CCl₂F | Φ-CClF₂ | Φ-CF₃ |
|---|---|---|---|---|---|---|
| [HF]₃- triéthylamine | 10h00 | 70°C | - | 86% | 8% | 0% |
| [HF]₁₀- pyridine 1 | 2h00 | 25°C | - | - | 92% | 8% |
| [HF]₁₀- pyridine 1 | 24h00 | 50°C | - | - | 15% | 84% |

### Exemple N°2 Echange Cl-F à partir du dichlorométhylbenzène

### 2a) Echange d'un atome de chlore

### Equation réactionnelle

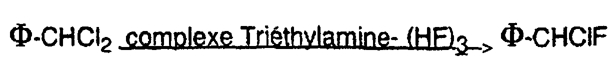

Mode opératoire utilisé:
0,35g (2,17 mmole) de dichloro-méthyl benzène est additionné à 4,6g (0,03 mole) de complexe [HF]₃- triéthylamine à 20°C.

Le milieu réactionnel est ensuite chauffé et agité à 70°C pendant 14h.

Le brut réactionnel est alors versé sur un mélange de CH₂Cl₂ (50 ml) et de glace (50g).
La phase organique est lavée 4 fois par 20 ml d'eau, séchée sur sulfate de magnésium. Le solvant (CH₂Cl₂) est évaporé et le résidu est analysé par chromatographie en phase gazeuse et les structures des produits formés sont identifiés par RMN ¹⁹F.
On obtient alors majoritairement le composé Φ-CHCIF avec une sélectivité de l'ordre de 40%.
Le taux de transformation du dichlorométhylbenzène est de 50%.

### 2b) Echange de 2 atomes de chlore

### Equations réactionnelles

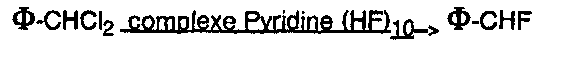

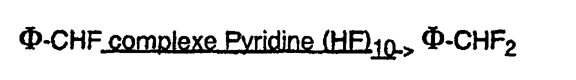

### Mode opératoire utilisé:

1.6 g (0,01 mole) du dichlorométhylbenzène est additionné à 11,2 g (0,04 mole) de complexe [HF]₁₀- pyridine 1 (préparé à partir de 77,3 g de pyridine et 200 g d'acide fluorhydrique anhydre) refroidi à 0°C.
Le milieu réactionnel est ensuite agité à 25°C pendant 14h.

Le brut réactionnel est alors versé sur un mélange de CH₂Cl₂ (200 ml) et de glace (150g).
La phase organique est lavée 4 fois par 50 ml d'eau, séchée sur sulfate de magnésium. Le solvant (CH₂Cl₂) est évaporé et le résidu est analysé par chromatographie en phase gazeuse et les structures des produits formés sont identifiés par RMN ¹⁹F.
On obtient alors majoritairement le composé Φ-CHF₂ avec une sélectivité de l'ordre de 70%.
Le taux de transformation du dichlorométhylbenzène est de 100%.

## Revendications

1. Procédé utile pour la synthèse de dérivés fluorés par échange entre un halogène de rang plus élevé et le fluor, **caractérisé par le fait qu'**il comporte une étape dans laquelle un substrat comportant au moins un carbone halogénophore d'hybridation sp3 porteur d'au moins deux halogènes dont au moins un est un halogène de nombre atomique supérieur à celui du fluor, lequel carbone halogénophore est relié à au moins un atome de faible hybridation porteur d'une insaturation, est soumis à l'action d'un réactif comportant au moins un composé défini choisi parmi ceux constitués par l'association d'une base de Bronstedt avec un nombre défini n d'acide fluorhydrique, n étant au moins égal à 3 et au plus à 20, avantageusement à 15, de préférence à 10, avec la condition que ledit carbone halogénophore ne porte pas de chalcogène. »

2. Procédé selon la revendication 1, **caractérisé par le fait que** l'éventuelle liaison résiduelle du carbone halogénophore étant avantageusement une liaison avec un groupe choisi parmi les groupes électroattracteurs par effet inductif, et parmi les groupes aryle.

3. Procédé selon les revendications 1 et 2, **caractérisé par le fait que** ledit atome de faible hybridation porteur d'une insaturation est un atome de carbone d'hybridation sp².

4. Procédé selon les revendications 1 à 3, **caractérisé par le fait que** ledit groupe choisi parmi les groupes électroattracteurs est un halogène.

5. Procédé selon les revendications 1 à 4, **caractérisé par le fait que** ledit carbone halogénophore porte au moins deux halogènes de nombre atomique supérieur à celui du fluor.

6. Procédé selon les revendications 1 à 5, **caractérisé par le fait que** ledit carbone halogénophore porte trois halogènes choisis parmi le chlore et le fluor.

7. Procédé selon les revendications 1 à 6, **caractérisé par le fait que** ledit atome de faible hybridation porteur d'une insaturation appartient à un cycle aromatique.

8. Procédé selon les revendications 1 à 7, **caractérisé par le fait que** ledit atome de faible hybridation porteur d'une insaturation appartient à un cycle aromatique riche ou peu appauvri en électron.

9. Procédé selon les revendications 1 à 8, **caractérisé par le fait que** ledit atome de faible hybridation porteur d'une insaturation appartient à un cycle aromatique comportant des hétéroatomes.

10. Procédé selon les revendications 1 à 9, **caractérisé par le fait que** ledit atome de faible hybridation porteur d'une insaturation appartient à un cycle aromatique à cinq ou à six chaînons.

11. Procédé selon les revendications 1 à 10, **caractérisé par le fait que** ledit atome de faible hybridation porteur d'une insaturation appartient à un cycle aromatique comportant cinq autres chaînons carbonés, eux-mêmes éventuellement substitués.

## Patentansprüche

1. Verfahren zur Synthese von Fluorderivaten durch Austausch zwischen einem Halogen mit höherer Rangordnung und Fluor, **dadurch gekennzeichnet, daß** es eine Stufe beinhaltet, bei der ein Substrat, welches zumindest einen Halogen-tragenden Kohlenstoff mit sp3-Hybridisierung aufweist, der Träger zumindest von zwei Halogenen ist, von denen zumindest eines ein Halogen mit höherer Atomzahl als derjenigen von Fluor ist, wobei der Halogen-tragende Kohlenstoff an zumindest ein eine Unsättigung tragendes Atom mit geringer Hybridisierung gebunden ist, der Einwirkung eines Reagenz unterzogen wird, das zumindest eine definierte Verbindung umfaßt, ausgewählt unter denjenigen, die gebildet werden durch Assoziation einer Brönstedt-Base mit einer definierten Anzahl n an Fluorwasserstoffsäure, wobei n zumindest 3 und höchstens 20, vorteilhafterweise 15, vorzugsweise 10, ist, unter der Voraussetzung, daß besagter Halogen-tragender Kohlenstoff kein Chalkogen enthält.

2. Verfahren nach Anspruch 1, **dadurch gekennzeichnet, daß** die etwaige verbleibende Bindung des Halogen-tragenden Kohlenstoffs vorteilhafterweise eine Bindung mit einer Gruppe, ausgewählt unter den durch induktiven Effekt Elektronen-anziehenden Gruppen und unter den Arylgruppen, ist.

3. Verfahren nach den Ansprüchen 1 und 2, **dadurch gekennzeichnet, daß** das Atom mit geringer Hybridiserung, das eine Unsättigung trägt, ein Kohlenstoffatom mit sp²-Hybridiserung ist.

4. Verfahren nach den Ansprüchen 1 bis 3, **dadurch gekennzeichnet, daß** die unter den Elektronen-anziehenden Gruppen ausgewählte Gruppe ein Halogen ist.

5. Verfahren nach den Ansprüchen 1 bis 4, **dadurch gekennzeichnet, daß** der Halogen-tragende Kohlenstoff zumindest zwei Halogene mit einer Atornzahl höher derjenigen von Fluor trägt.

6. Verfahren nach den Ansprüchen 1 bis 5, **dadurch gekennzeichnet, daß** der Halogen-tragende Kohlenstoff drei Halogene, ausgewählt unter Chlor und Fluor, trägt.

7. Verfahren nach den Ansprüchen 1 bis 6, **dadurch gekennzeichnet, daß** das Atom mit geringer Hybridisierung, welches Träger einer Unsättigung ist, einem aromatischen Ring zugehört.

8. Verfahren nach den Ansprüchen 1 bis 7, **dadurch gekennzeichnet, daß** das Atom mit geringer Hybridiserung, das Träger einer Unsättigung ist, einem elektronenreichen oder elektronenarmen aromatischen Ring zugehört.

9. Verfahren nach den Ansprüchen 1 bis 8, **dadurch gekennzeichnet, daß** das Atom mit geringer Hybridiserung, das Träger einer Unsättigung ist, einem Heteroatome enthaltenden aromatischen Ring zugehört.

10. Verfahren nach den Ansprüchen 1 bis 9, **dadurch gekennzeichnet, daß** das Atom mit geringer Hybridiserung, das Träger einer Unsättigung ist, einem aromatischen Ring mit 5 oder 6 Ringgliedern zugehört.

11. Verfahren nach den Ansprüchen 1 bis 10, **dadurch gekennzeichnet, daß** das Atom mit geringer Hybridiserung, das Träger einer Unsättigung ist, einem aromatischen Ring zugehört, der fünf weitere kohlenstoffhaltige Ringglieder enthält, die ihrerseits gegebenenfalls substituiert sind.

## Claims

1. Process that is useful for synthesizing fluoro derivatives by exchange between a halogen of a higher row and fluorine, **characterized in that** it comprises a step in which a substrate comprising at least one halophoric carbon of sp³ hybridization bearing at least two halogens, at least one of which is a halogen with an atomic number higher than that of fluorine, this halophoric carbon being linked to at least one atom of low hybridization bearing an unsaturation, is subjected to the action of a reagent comprising at least one defined compound chosen from those consisting of the combination of a Brönstedt base with a defined number n of hydrofluoric acid, n being at least equal to 3 and not more than 20, advantageously 15 and preferably 10, with the condition that the said halophoric carbon does not bear a chalcogen.

2. Process according to claim 1, **characterized in that** the optional residual bond of the halophoric carbon is advantageously a bond with a group chosen from groups that are electron-withdrawing by an inductive effect, and from aryl groups.

3. Process according to claims 1 and 2, **characterized in that** the said atom of low hybridization bearing an unsaturation is a carbon atom of sp² hybridization.

4. Process according to claims 1 to 3, **characterized in that** the said group chosen from electron-withdrawing groups is a halogen.

5. Process according to claims 1 to 4, **characterized in that** the said halophoric carbon bears at least two halogens with an atomic number higher than that of fluorine.

6. Process according to claims 1 to 5, **characterized in that** the said halophoric carbon bears three halogens chosen from chlorine and fluorine.

7. Process according to claims 1 to 6, **characterized in that** the said atom of low hybridization bearing an unsaturation belongs to an aromatic ring.

8. Process according to claims 1 to 7, **characterized in that** the said atom of low hybridization bearing an unsaturation belongs to an aromatic ring that is electron-rich or not particularly electron-poor.

9. Process according to claims 1 to 8, **characterized in that** the said atom of low hybridization bearing an unsaturation belongs to an aromatic ring comprising hetero atoms.

10. Process according to claims 1 to 9, **characterized in that** the said atom of low hybridization bearing an unsaturation belongs to a five-membered or six-membered aromatic ring.

11. Process according to claims 1 to 10, **characterized in that** the said atom of low hybridization bearing an unsaturation belongs to an aromatic ring comprising five other carbon ring members, which are themselves optionally substituted.
